# EUROPEAN PATENT SPECIFICATION

(11) **EP 2 421 489 B1**
(45) Date of publication and mention of the grant of the patent: **03.10.2012**
(21) Application number: 10717223.1
(22) Date of filing: 20.04.2010
(51) Int. Cl.: A61F 7/02, A61M 5/44, A61J 1/16

(54) **APPLIANCE FOR HEATING AND MAINTAINING THE TEMPERATURE OF MEDICAL, DIAGNOSTIC AND PHYSIOLOGICAL FLUIDS**
GERÄT ZUM ERWÄRMEN UND AUFRECHTERHALTEN DER TEMPERATUR VON MEDIZINISCHEN, DIAGNOSTISCHEN UND PHYSIOLOGISCHEN FLÜSSIGKEITEN
APPAREIL POUR CHAUFFER ET MAINTENIR LA TEMPÉRATURE DE FLUIDES MÉDICAUX, DE DIAGNOSTIC ET PHYSIOLOGIQUES

(30) Priority: 24.04.2009 IT MO20090098
(43) Date of publication of application: 29.02.2012
(73) Proprietor: Azzolini, Graziano, 41032 Cavezzo (MO) (IT)
(72) Inventor: Azzolini, Graziano, 41032 Cavezzo (MO) (IT)
(74) Representative: Brunacci, Marco
(86) International application number: PCT/IB2010/000878
(87) International publication number: WO 2010/122399

(56) References cited:
- WO-A1-98/45658
- US-A- 5 572 873
- US-B1- 6 384 380

## Description

### Technical Field

The present invention relates to an appliance for heating and maintaining the temperature of medical, diagnostic and physiological fluids.

### Background Art

With reference to the medical field, the need is known and felt to heat medical, diagnostic or physiological fluids to body temperature and maintain them at such temperature.

In radiology, e.g., it is known that the increasingly greater efficiency of machines for carrying out CAT scans (Computerized Axial Tomography) is due, besides to growing technological developments, also to the use of more efficient contrast agents to be administered to the patient.

In particular, such contrast agents comprise increasingly bigger percentages of substances such as iodine, which allow a clearer identification of details in the area examined by CAT scan.

An increase in the percentage of such substances however, makes the contrast agents increasingly more viscous and this affects the pressure of administering to the patient according to the flow.

Pressure must therefore necessarily be kept lower in order to restrict the operation of the self-adjustment means conventionally fitted to injection machines suitable for administering contrast agents to the patient.

These administering self-adjustment means in fact operate when the pressure is too high to reduce the flow of the administered contrast agents or, also, to block administration.

Therefore, the heating of the contrast agents before use is of major importance, in particular because it allows considerably reducing the viscosity of such agents and, consequently, increasing the flow of administration to the patient. Generally speaking, the medical, diagnostic and physiological fluids have to be heated gradually, in predefined times and ways so as not to alter their specific properties and, once they have reached body temperature of around 37°C, must be rigorously kept at that temperature until the time of administration to a patient.

The medical, diagnostic and physiological fluids are generally available in special containers such as bottles made of glass or polymer material and bags made of flexible plastic material.

To heat and keep the temperature of these fluids in a medical field, the use is known of appliances, commonly known as thermostatic cells, having a housing compartment suitable for accommodating the containers and having heating means suitable for heating the fluids inside the containers up to the required temperature before administering to patients. An example of known appliance is given by the United States Patent US 6, 384, 380 B1.

These known appliances do however have a number of drawbacks.

These appliances in fact are unable to ensure that constant and uniform temperature is maintained inside the entire housing compartment of the fluid containers.

Consequently, whenever it becomes necessary to administer the fluid to a patient, the fluids inside different containers have different temperatures the one from the other.

### Object of the Invention

The main aim of the present invention is to provide an appliance for heating and maintaining the temperature of medical, diagnostic and physiological fluids that allows heating a fluid and maintaining it at a constant and uniform temperature. Another object of the present invention is to provide an appliance for heating and maintaining the temperature of medical, diagnostic and physiological fluids that allows housing several containers of a fluid and heating and maintaining substantially at a same temperature the fluid inside each of the containers. Another object of the present invention is to provide an appliance for heating and maintaining the temperature of medical, diagnostic and physiological fluids which allows overcoming the mentioned drawbacks of the state of the art within the ambit of a simple, rational, easy, effective to use and low cost solution.

The above objects are achieved by the present appliance for heating and maintaining the temperature of medical, diagnostic and physiological fluids, comprising a bearing structure, at least a compartment inside said bearing structure having at least an opening for accessing from outside and suitable for housing at least a container with a medical, diagnostic or physiological fluid to be heated, heating means arranged inside said compartment and control means for controlling said heating means for achieving and maintaining a predefined temperature inside said compartment, characterised by the fact that it comprises at least a blowing fan and at least a suction fan arranged inside said compartment and suitable for blowing and suctioning air for the uniform distribution of said predefined temperature inside said compartment.

### Brief Description of the Drawings

Other characteristics and advantages of the present invention will become more evident from the description of a preferred, but not sole, embodiment of an appliance for heating and maintaining the temperature of medical, diagnostic and physiological fluids, illustrated purely as an example but not limited to the annexed drawings in which:

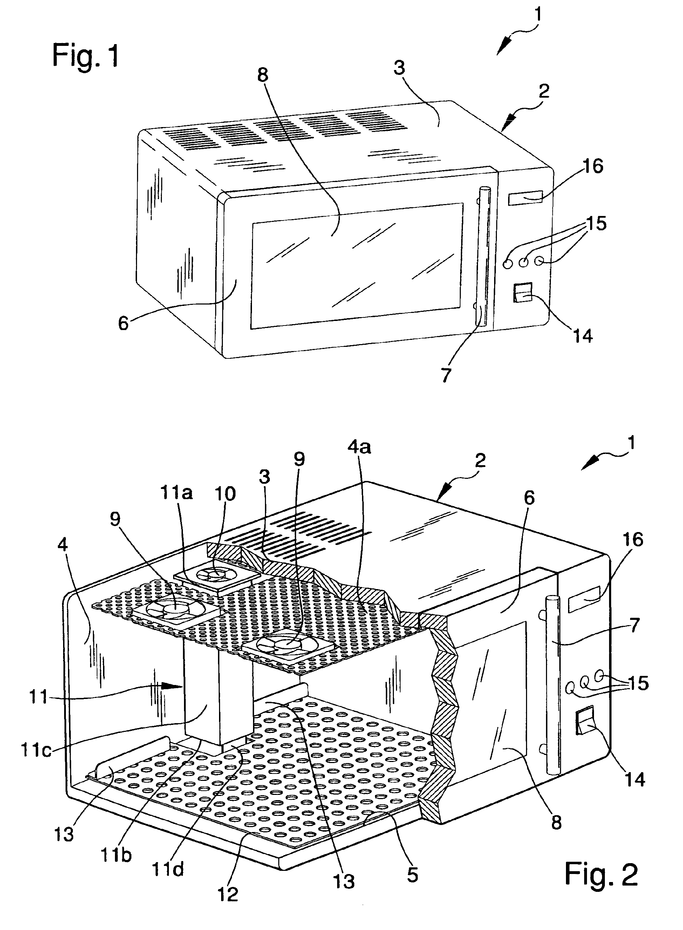

figure 1 is an axonometric view of the appliance according to the invention;
figure 2 is an axonometric and partially sectioned view of the appliance according to the invention;
figure 3 is a front perspective view of the appliance according to the invention;
figure 4 is an axonometric view of the appliance according to the invention having supporting means for the fluid bags.

### Embodiments of the Invention

With particular reference to such figures, globally indicated by 1 is an appliance for heating and maintaining the temperature of medical, diagnostic and physiological fluids.

The appliance 1, e.g., can be of the type usable in radiology to heat contrast agents to be administered to a patient before a CAT scan.

The appliance 1 comprises a bearing structure 2, having an outer covering wrapping 3 preferably in stainless steel and an inner compartment 4, delimited by stainless-steel walls, suitable for housing one or more containers of a medical, diagnostic or physiological fluid to be heated.

In particular, the appliance 1 has, on the front, an opening 5 for accessing the compartment 4 from outside and a door 6 hinged revolving to the bearing structure 2 and positionable in correspondence to the opening 5 for hermetically closing the compartment 4.

Usefully, the door 6 can have a grip handle 7 and a transparent portion 8 made of glass to allow viewing the containers arranged inside the compartment 4.

Inside the compartment 4 are heating means of the type, e.g., of one or more heating elements suitable arranged and shielded, and control means for controlling such heating means for achieving and maintaining a predefined temperature inside the compartment 4. Such heating means are not shown in the illustrations inasmuch as of known type.

Preferably, the control means are composed of an electronic device and of a respective software program suitable for piloting the heating means to maintain a predefined temperature equal to body temperature (about 37°C).

In particular, the control means can comprise an electronic device of the type of a microprocessor or the like and a relative implementation software of a PID (proportional-integral-derivative) type negative feedback control system. Advantageously, the appliance 1 comprises at least a blowing fan 9 and at least a suction fan 10 arranged inside the compartment 4 and suitable for blowing and suctioning air for the uniform distribution of the temperature inside the compartment itself.

With particular, but not sole reference to the embodiment shown in the illustrations, the appliance 1 comprises two pairs of blowing fans 9 arranged in correspondence to two opposite portions respectively on an upper wall 4a of the compartment 4.

A suction fan 10 is arranged in correspondence to a central portion of this upper wall 4a of the compartment 4, between the two pairs of blowing fans 9.

The appliance 1 also comprises a suction manifold 11, with a rectangular-section tubular shape, which has an open extremity 11a connected hermetically to the suction fan 10, a suction mouth 11b arranged in the proximity of the bottom of the compartment 4 and a connection duct 11c between the open extremity 11a and the suction mouth 11b which extends vertically inside the compartment 4.

In particular, the connection duct 11c can be fastened to the bottom of the compartment 4, in correspondence to the suction mouth 11b, by means of one or more specific brackets 11d.

A different number and a different arrangement cannot however be ruled out of the blowing fans 9 and of the suction fan 10, as well as a different shape and a different arrangement of the suction manifold 11.

Such arrangement of the blowing fans 9 and of the suction fan 10, as well as the presence of the suction manifold 11, allow optimising the ventilation inside the compartment 4, so as to obtain a uniform distribution of the temperature inside the compartment itself with a tolerance of less than ± 1 °C.

Usefully, the appliance 1 comprises a removable shelf 12 positionable on the bottom of the compartment 4 and suitable for accommodating by resting the containers of the fluid to be heated.

Preferably, the shelf 12 is made of stainless steel and has a plurality of holes for the flow of air suitable for ensuring a better ventilation around the containers. Usefully, the shelf 12 comprises a pair of stop elements 13 suitable for preventing the contact between the containers and the wall of the compartment opposite the opening 5.

The stop elements 13, in particular, are made up of two barriers fastened to the shelf 12 which extend along the shelf side by side with each other, in proximity of the wall of the compartment 4 opposite the access opening 5.

The use of a different number of stop elements 13 cannot however be ruled out, in different positions on the shelf 12 and of different shape.

The appliance 1 further comprises one or more temperature probes, not shown in the illustrations being of known type, arranged inside the compartment 4, associated with the control means and used to continuously check the temperature inside the compartment itself.

Usefully, a further detection sensor can be provided for detecting the opening/closing of the door 6, this too operatively associated with the control means.

The appliance 1 comprises interface means, operatively associated with the control means and provided with sound and/or visual signalling means, which are usable by an operator for the control and management of the appliance itself.

With particular reference to the embodiment of the appliance 1 shown in the above illustrations, the interface means comprise a switch 14 for switching the appliance itself on and off, a series of control buttons 15 and a display screen 16 for displaying information such as, e.g., the current internal temperature, error signals, etc.

Usefully, the appliance 1 can comprise a timer associated with the control means and programmable by means of the interface means for the automatic switch-on of the heating means. In point of fact, the timer can be suitably programmed by an operator, specifying when the fluid has to be ready for use. This way, the heating means are switched on in advance, so as to ensure the fluid is available at the correct temperature at the time when it has to be used. The medical, diagnostic and physiological fluids to be heated are generally available in special containers such as bottles made of glass or polymer material or, also, in flexible plastic bags.

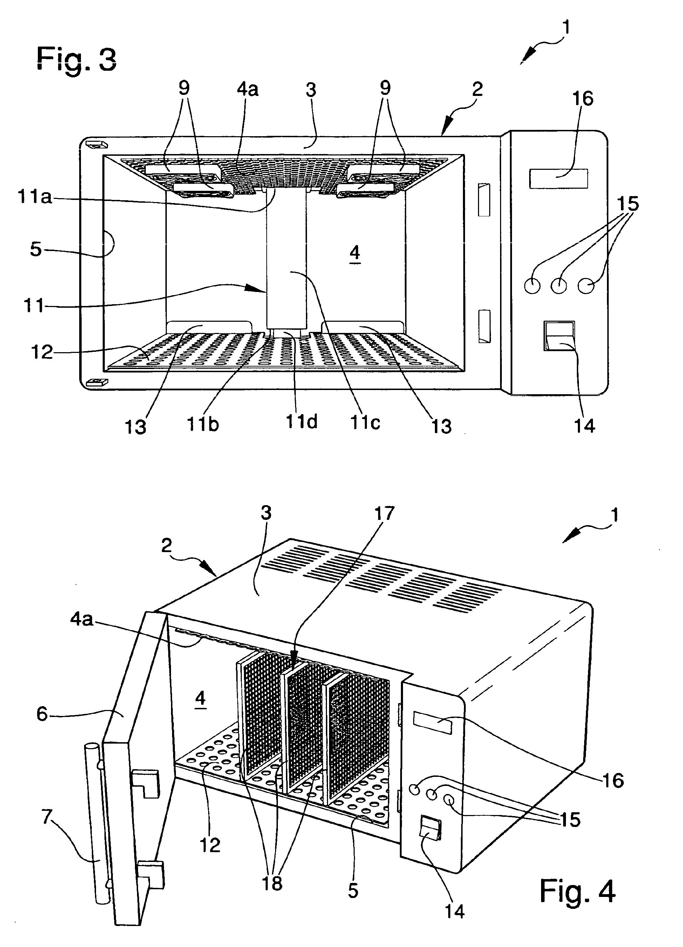

In this latter case, as shown in figure 4, the appliance 1 can be provided with a rack 17 suitable for accommodating and supporting the fluid bags vertically.

In particular, this rack 17 can be made by means of a series of grilles 18, suitably perforated to allow air to flow through, arranged vertically and positioned one alongside the other.

Rows of several fluid bags can therefore be arranged inside the compartment 4, vertically, between one grille 18 and the next.

It has in practice been found how the described invention achieves the intended objects.

In particular, the fact is underlined that the appliance according to the invention, in particular the presence of the blowing fans and of the suction fan, allows heating and maintaining a constant and uniform temperature inside the entire compartment.

This way, furthermore, for each of the containers housed inside the compartment, the fluid is heated at the same predefined temperature, with a tolerance below ± 1°C.

Another advantage stems from the fact that the absence of external thermostats suitable for regulating the temperature prevents errors on the part of the operator in charge of fluid heating.

## Claims

1. Appliance (1) for heating and maintaining the temperature of medical, diagnostic and physiological fluids, comprising a bearing structure (2), at least a compartment (4) inside said bearing structure (2) having at least an opening (5) for accessing from outside and suitable for housing at least a container with a medical, diagnostic or physiological fluid to be heated, heating means arranged inside said compartment (4) and control means for controlling said heating means for achieving and maintaining a predefined temperature inside said compartment (4), **characterised by** the fact that it comprises at least a blowing fan (9) and at least a suction fan (10) arranged inside said compartment (4) and suitable for blowing and suctioning air for the uniform distribution of said predefined temperature inside said compartment (4).

2. Appliance (1) according to the claim 1, **characterised by** the fact that said blowing fan (9) is arranged in correspondence to an upper portion of said compartment (4).

3. Appliance (1) according to one or more of the preceding claims, **characterised by** the fact that said suction fan (10) is arranged in correspondence to an upper portion of said compartment (4).

4. Appliance (1) according to one or more of the preceding claims, **characterised by** the fact that it comprises a suction fan (10) arranged in correspondence to a substantially central portion of an upper wall of said compartment (4) and two pairs of blowing fans (9) arranged at the sides of said suction fan (10), on said upper wall.

5. Appliance (1) according to one or more of the preceding claims, **characterised by** the fact that it comprises at least a suction manifold (11) having at least an open extremity (11a) associated with said suction fan (10), at least a suction mouth (11b) arranged inside said compartment (4) and at least a connection duct (11c) between said open extremity (11a) and said suction mouth (11b).

6. Appliance (1) according to one or more of the preceding claims, **characterised by** the fact that said connection duct (11c) of the suction manifold (11) extends substantially vertically inside said compartment (4), with said open extremity (11a) associated with said suction fan (10) in correspondence to an upper wall of said compartment (4) and said suction mouth (11b) arranged in the proximity of the bottom of said compartment (4).

7. Appliance (1) according to one or more of the preceding claims, **characterised by** the fact that it comprises at least a removable shelf positionable inside said compartment (4) and suitable for accommodating by resting said fluid container.

8. Appliance (1) according to one or more of the preceding claims, **characterised by** the fact that said shelf (12) is positionable at the bottom of said compartment (4).

9. Appliance (1) according to one or more of the preceding claims, **characterised by** the fact that said shelf (12) comprises a plurality of holes for the flow of air.

10. Appliance (1) according to one or more of the preceding claims, **characterised by** the fact that said shelf (12) comprises at least a stop element (13) of said container.

11. Appliance (1) according to one or more of the preceding claims, **characterised by** the fact that said stop element (13) comprises at least a barrier (13) which extends along at least a section of said shelf (12).

12. Appliance (1) according to one or more of the preceding claims, **characterised by** the fact that it comprises at least a pair of said barriers (13) arranged side by side with each other on said shelf (12) in the proximity of the wall of said compartment (4) substantially opposite said access opening (5).

13. Appliance (1) according to one or more of the preceding claims, **characterised by** the fact that it comprises at least a rack (17) positionable inside said compartment (4) and suitable for accommodating and supporting vertically bags made of polymer material containing said fluid.

14. Appliance (1) according to one or more of the preceding claims, **characterised by** the fact that said rack (17) comprises at least two grilles (18) arranged vertically and side by side with each other.

15. Appliance (1) according to one or more of the preceding claims, **characterised by** the fact that it comprises at least a temperature probe arranged inside said compartment (4) and operatively associated with said control means.

## Patentansprüche

1. Gerät (1) zum Erwärmen und Aufrechterhalten der Temperatur von medizinischen, diagnostischen und physiologischen Flüssigkeiten, mit einer Aufnahmestruktur (2), mindestens einem Abteil (4) innerhalb der Aufnahmestruktur (2), mit mindestens einer Öffnung (5) für den zugang von außen und geeignet, mindestens einen Behälter mit einer zu erwärmenden medizinischen, diagnostischen oder physiologischen Flüssigkeit aufzunehmen, mit innerhalb des Abteils (4) angeordneten Heizeinrichtungen und mit Regelungseinrichtungen zum Regeln der Heizeinrichtungen, um eine vorgegebene Temperatur innerhalb des Abteils (4) zu erzielen und aufrechtzuerhalten, **dadurch gekennzeichnet, dass** es mindestens einen Blaslüfter (9) und mindestens einen Sauglüfter (10) aufweist, die innerhalb des Abteils (4) angeordnet und geeignet sind, Luft für die gleichmäßige Verteilung der vorgegebenen Temperatur innerhalb des Abteils (4) zu blasen und zu saugen.

2. Gerät (1) nach Anspruch 1, **dadurch gekennzeichnet, dass** der Blaslüfter (9) in Entsprechung zu einem oberen Abschnitt des Abteils (4) angeordnet ist.

3. Gerät (1) nach einem oder mehr der vorangegangenen Ansprüche, **dadurch gekennzeichnet, dass** der Sauglüfter (10) in Entsprechung zu einem oberen Abschnitt des Abteils (4) angeordnet ist.

4. Gerät (1) nach einem oder mehr der vorangegangenen Ansprüche, **dadurch gekennzeichnet, dass** es einen in Entsprechung zu einem im Wesentlichen mittigen Abschnitt einer oberen Wand des Abteils (4) angeordneten Sauglüfter (10) und zwei an den Seiten des Sauglüfters (10) an der oberen Wand angeordnete Paare Blaslüfter (9) aufweist.

5. Gerät (1) nach einem oder mehr der vorangegangenen Ansprüche, **dadurch gekennzeichnet, dass** es mindestens eine Saugsammelleitung (11) mit mindestens einem mit dem Sauglüfter (10) verbundenen, offenen Ende (11a), mindestens einer innerhalb des Abteils (4) angeordneten Saugmündung (11b) und mindestens einer Verbindungsleitung (11c) zwischen dem offenen Ende (11a) und der Saugmündung (11b) aufweist.

6. Gerät (1) nach einem oder mehr der vorangegangenen Ansprüche, **dadurch gekennzeichnet, dass** die Verbindungsleitung (11c) der Saugsammelleitung (11) im Wesentlichen vertikal innerhalb des Abteils (4) verläuft, wobei das offene Ende (11a) mit dem Sauglüfter (10) in Entsprechung zu einer oberen Wand des Abteils (4) verbunden ist und die Saugmündung (11b) in der Nähe der Unterseite des Abteils (4) angeordnet ist.

7. Gerät (1) nach einem oder mehr der vorangegangenen Ansprüche, **dadurch gekennzeichnet, dass** es mindestens einen entfernbaren Tragrost aufweist, der innerhalb des Abteils (4) positionierbar und geeignet ist, den Flüssigkeitsbehälter durch Aufliegen aufzunehmen.

8. Gerät (1) nach einem oder mehr der vorangegangenen Ansprüche, **dadurch gekennzeichnet, dass** der Tragrost (12) auf dem Boden des Abteils (4) positionierbar ist.

9. Gerät (1) nach einem oder mehr der vorangegangenen Ansprüche, **dadurch gekennzeichnet, dass** der Tragrost (12) eine Vielzahl von Löchern für den Luftstrom aufweist.

10. Gerät (1) nach einem oder mehr der vorangegangenen Ansprüche, **dadurch gekennzeichnet, dass** der Tragrost (12) mindestens ein Stoppelement (13) für den Behälter aufweist.

11. Gerät (1) nach einem oder mehr der vorangegangenen Ansprüche, **dadurch gekennzeichnet, dass** das Stoppelement (13) mindestens eine Barriere (13) aufweist, die entlang mindestens einem Abschnitt des Tragrosts (12) verläuft.

12. Gerät (1) nach einem oder mehr der vorangegangenen Ansprüche, **dadurch gekennzeichnet, dass** es mindestens ein Paar der Barrieren (13), die Seite an Seite beieinander auf dem Tragrost (12) in der Nähe der Wand des Abteils (4) im Wesentlichen gegenüber der Zugangsöffnung (5) angeordnet sind, aufweist.

13. Gerät (1) nach einem oder mehr der vorangegangenen Ansprüche, **dadurch gekennzeichnet, dass** es mindestens ein Gestell (17) aufweist, das innerhalb des Abteils (4) positionierbar und geeignet ist, aus Polymermaterial hergestellte Beutel, die jene Flüssigkeit enthalten, aufzunehmen und vertikal zu stützen.

14. Gerät (1) nach einem oder mehr der vorangegangenen Ansprüche, **dadurch gekennzeichnet, dass** das Gestell (17) mindestens zwei vertikal Seite an Seite beieinander angeordnete Trenngitter (18) aufweist.

15. Gerät (1) nach einem oder mehr der vorangegangenen Ansprüche, **dadurch gekennzeichnet, dass** es mindestens eine innerhalb des Abteils (4) angeordnete und mit den Regeleinrichtungen funktional verbundene Temperatursonde aufweist.

## Revendications

1. Dispositif (1) de chauffage et de maintien de la température de fluides médicaux, de diagnostic et physiologiques, comprenant une structure porteuse (2), au moins un compartiment (4) à l'intérieur de ladite structure porteuse (2) ayant au moins une ouverture (5) pour un accès depuis l'extérieur et apte à recevoir au moins un récipient contenant un fluide médical, de diagnostic ou physiologique à chauffer, des moyens de chauffage ménagés à l'intérieur dudit compartiment (4) et des moyens de contrôle pour contrôler lesdits moyens de chauffage afin d'atteindre et de maintenir une température prédéfinie à l'intérieur dudit compartiment (4), ***caractérisé par le fait qu'*il** comprend au moins un ventilateur soufflant (9) et au moins un ventilateur aspirant (10) disposés à l'intérieur dudit compartiment (4) et apte à souffler et à aspirer de l'air pour la distribution uniforme de ladite température prédéfinie à l'intérieur dudit compartiment (4).

2. Dispositif (1) selon la revendication 1, ***caractérisé par le fait que*** ledit ventilateur soufflant (9) est disposé en correspondance avec une portion supérieure dudit compartiment (4).

3. Dispositif (1) selon l'une ou plusieurs des revendications précédentes, ***caractérisé par le fait que*** ledit ventilateur aspirant (10) est disposé en correspondance avec une portion supérieure dudit compartiment (4).

4. Dispositif (1) selon l'une ou plusieurs des revendications précédentes, ***caractérisé par le fait qu'il*** comprend un ventilateur aspirant (10) disposé en correspondance avec une portion sensiblement centrale d'une paroi supérieure dudit compartiment (4) et deux paires de ventilateurs soufflants (9) disposés sur les côtés dudit ventilateur aspirant (10), sur ladite paroi supérieure.

5. Dispositif (1) selon l'une ou plusieurs des revendications précédentes, ***caractérisé par le fait qu'il*** comprend au moins un collecteur d'admission (11) ayant au moins une extrémité ouverte (11a) associée audit ventilateur aspirant (10), au moins une bouche d'aspiration (11b) située à l'intérieur dudit compartiment (4) et au moins un conduit de liaison (11c) entre ladite extrémité ouverte (11a) et ladite bouche d'aspiration (11b).

6. Dispositif (1) selon l'une ou plusieurs des revendications précédentes, ***caractérisé par le fait que*** ledit conduit de liaison (11c) du collecteur d'admission (11) s'étend sensiblement verticalement à l'intérieur dudit compartiment (4), avec ladite extrémité ouverte (11a) associée audit ventilateur aspirant (10) en correspondance avec une paroi supérieure dudit compartiment (4) et ladite bouche d'aspiration (11b) située à proximité du fond dudit compartiment (4).

7. Dispositif (1) selon l'une ou plusieurs des revendications précédentes, ***caractérisé par le fait qu'***il comprend au moins un rayonnage amovible positionnable à l'intérieur dudit compartiment (4) et apte à accueillir en appui ledit récipient pour le fluide.

8. Dispositif (1) selon l'une ou plusieurs des revendications précédentes, ***caractérisé par le fait que*** ledit rayonnage (12) est positionnable au fond dudit compartiment (4).

9. Dispositif (1) selon l'une ou plusieurs des revendications précédentes, ***caractérisé par le fait que*** ledit rayonnage (12) comprend une pluralité d'orifices pour le passage de l'air.

10. Dispositif (1) selon l'une ou plusieurs des revendications précédentes, ***caractérisé par le fait que*** ledit rayonnage (12) comprend au moins un élément de retenue (13) dudit récipient.

11. Dispositif (1) selon l'une ou plusieurs des revendications précédentes, ***caractérisé par le fait que*** ledit élément de retenue (13) comprend au moins une barrière qui s'étend le long d'au moins une section dudit rayonnage (12).

12. Dispositif (1) selon l'une ou plusieurs des revendications précédentes, ***caractérisé par le fait qu'il*** comprend une paire desdites barrières (13) disposées côte à côte sur ledit rayonnage (12) à proximité de la paroi dudit compartiment (4) sensiblement opposée à ladite ouverture d'accès (5).

13. Dispositif (1) selon l'une ou plusieurs des revendications précédentes, ***caractérisé par le fait qu'il*** comprend au moins un râtelier (17) positionnable à l'intérieur dudit compartiment (4) et apte à recevoir et supporter verticalement des poches en matériau polymère contenant ledit fluide.

14. Dispositif (1) selon l'une ou plusieurs des revendications précédentes, ***caractérisé par le fait que*** ledit râtelier (17) comprend au moins deux grilles (18) placées verticalement et côte à côte.

15. Dispositif (1) selon l'une ou plusieurs des revendications précédentes, ***caractérisé par le fait qu'il*** comprend au moins une sonde de température placée à l'intérieur dudit compartiment (4) et associée opérationnellement auxdits moyens de contrôle.
